# FASCICULE DE BREVET EUROPEEN

(11) **EP 4 373 888 B1**
(45) Date de publication et mention de la délivrance du brevet: **03.09.2025**
(21) Numéro de dépôt: 22751397.5
(22) Date de dépôt: 20.07.2022
(51) Int. Cl.: C08L 67/04, C12N 9/96, C08L 5/00, C08L 3/00, C08L 1/00, B32B 27/36, C08J 5/18, C08J 11/10, C08J 3/22, C08K 3/26, C12N 9/16, C12N 9/18

(54) **PROCÉDÉ DE PRÉPARATION D'UN MÉLANGE MAÎTRE ENZYMÉ**
VERFAHREN ZUR HERSTELLUNG EINES ENZYM-MASTERBATCHES
METHOD FOR PREPARING AN ENZYME MASTERBATCH

(30) Priorité: 20.07.2021 FR 2107809
(43) Date de publication de la demande: 29.05.2024
(73) Titulaire: Carbiolice, 63100 Clermont-Ferrand (FR)
(72) Inventeur: GUILLAUMONT, Chloé, 63200 CHAMBARON SUR MORGE (FR); ARNAULT, Clémentine, 63100 CLERMONT FERRAND (FR); CHARPENTIER, Yannick, 63260 AIGUEPERSE (FR)
(74) Mandataire: Icosa
(86) Numéro de dépôt international: PCT/EP2022/070302
(87) Numéro de publication internationale: WO 2023/001872

(56) Documents cités:
- WO-A1-2016/198652
- WO-A1-2019/043134
- WO-A1-2020/193770
- FR-A1- 2 984 354

## Description

### DOMAINE DE L'INVENTION

La présente invention concerne un procédé de préparation d'un mélange maître (ou « masterbatch ») comprenant un polysaccharide, des enzymes et un polymère à bas point de fusion dans un mélangeur. Ce mélange maître est notamment utilisé pour la fabrication d'articles de matière plastique biodégradables.

### ETAT DE LA TECHNIQUE

Des procédés de préparation de matières plastiques à base de polyesters biodégradables et biosourcés ont été développés afin de répondre aux enjeux écologiques. Ces produits de matière plastique, synthétisés à partir d'amidon ou de dérivés de l'amidon et de polyester, sont utilisés pour la fabrication d'articles ayant une courte durée de vie, tels que les sacs en plastique, les emballages alimentaires, les bouteilles, les films d'enrubannage, etc.

Ces compositions de plastique contiennent généralement du polyester et des farines issues de diverses céréales (US 5,739,244; US 6,176,915; US 2004/0167247; WO 2004/113433; FR 2 903 042; FR 2 856 405).

Dans le but de contrôler la dégradation de ces produits de matière plastique, l'ajout d'additif(s) comme des charges minérales (WO 2010/041063) et/ou d'entités biologiques ayant une activité de dégradation de polyester (WO 2013/093355; WO 2016/198652; WO 2016/198650; WO 2016/146540; WO 2016/062695) a été proposé.

Les articles de matière plastique biodégradable comprenant des entités biologiques, plus particulièrement des enzymes dispersées dans un polymère, présentent ainsi une meilleure biodégradabilité par rapport aux produits de matière plastique dépourvus de ces enzymes.

Des procédés de préparation de ces matières plastiques enzymées ont précédemment été décrits, cependant des problèmes liés à l'homogénéité et à la rugosité peuvent apparaître et se répercuter sur les propriétés physiques du produit. Par exemple, la présence d'agrégats d'enzymes entraine une rugosité plus importante, l'esthétisme du produit est diminué et les propriétés physiques et mécaniques sont altérées.

Une première amélioration a été apportée en ajoutant au polymère support l'enzyme préalablement mélangée avec un polysaccharide et un solvant dans une seule formulation liquide (WO 2019/043145, WO 2019/043134).

L'objet de l'invention est de faciliter et sécuriser la mise en oeuvre industrielle du procédé de préparation du mélange maître, notamment en simplifiant les outils nécessaires à sa mise en oeuvre tout en conservant, voire améliorant, leurs performances en termes de préservation de l'activité des enzymes après formulation, et de capacité à être employés dans la préparation d'articles finaux.

La présente invention décrit un procédé de préparation d'un mélange maître, qui utilisé dans la fabrication de produits de matière plastique comprenant des enzymes dispersées dans un polymère, permet d'améliorer la dispersion des enzymes dans le composé final ainsi que le taux de biodégradabilité de la matière plastique sans modifier les propriétés mécaniques du produit.

### EXPOSE DE L'INVENTION

La présente invention concerne un procédé de préparation d'un mélange maître comprenant un polysaccharide, des enzymes et un polymère support dans un mélangeur, ledit procédé comprenant les étapes suivantes de :
a) introduction séparée et simultanée a1) des enzymes en solution, a2) du polysaccharide et a3) du polymère support,
b) leur mélange à une température à laquelle le polymère support est partiellement ou totalement fondu, et
c) récupération du mélange maître.

L'invention concerne aussi les mélanges maîtres ainsi obtenus et des articles de matière plastique obtenus en mélangeant le mélange maître avec un polymère ou un mélange de polymères comprenant un polymère capable d'être dégradé par les enzymes du mélange maître. Elle concerne en particulier un procédé de préparation d'un article de matière plastique comprenant un polymère susceptible d'être dégradé par des enzymes et des enzymes capables de dégrader ledit polymère, comprenant une étape de mélange du mélange maître selon l'invention avec ledit polymère, seul ou en mélange.

### DESCRIPTION DETAILLEE DE L'INVENTION

La présente invention concerne un procédé de préparation d'un mélange maître comprenant un polysaccharide, des enzymes capables de dégrader des polyesters et un polymère support dans un mélangeur, ledit procédé comprenant les étapes suivantes de :
a) introduction séparée et simultanée d'une formulation liquide d'enzymes, du polysaccharide et du polymère support,
b) et leur mélange à une température à laquelle le polymère support est partiellement ou totalement fondu, et
c) récupération du mélange maître.

Sauf indication contraire, les pourcentages sont donnés en poids par rapport au poids total de la composition à laquelle ils se réfèrent.

Tel qu'il est utilisé ici, le terme «polysaccharides» fait référence à des molécules composées de longues chaînes d'unités monosaccharides liées ensemble par des liaisons glycosidiques. La structure des polysaccharides peut être linéaire à fortement ramifiée. Les exemples incluent les polysaccharides de stockage tels que l'amidon et le glycogène, et les polysaccharides structurels tels que la cellulose et la chitine. Les polysaccharides comprennent les polysaccharides natifs ou les polysaccharides chimiquement modifiés par réticulation, oxydation, acétylation, hydrolyse partielle, etc.

Les polymères glucidiques peuvent être classés en fonction de leur source (marine, végétale, microbienne ou animale), de la structure (linéaire, ramifiée) et/ou un comportement physique (tel que la désignation comme gomme ou hydrocolloïde qui fait référence à la propriété que ces polysaccharides hydratent dans l'eau chaude ou froide pour former des solutions ou des dispersions visqueuses à faible concentration en gomme ou hydrocolloïde).

Dans le cadre de l'invention, les polysaccharides peuvent être classés selon la classification décrite dans «Technologies d'encapsulation pour ingrédients actifs alimentaires et transformation alimentaire - Chapitre 3 - Matériaux pour encapsulation - Christine Wandrey, Artur Bartkowiak et Stephen E. Harding» :
- Amidon et dérivés, tels que l'amylose, l'amylopectine, la maltodextrine, les sirops de glucose, la dextrine, la cyclodextrine
- Cellulose et dérivés, tels que la méthylcellulose, l'hydroxypropylméthylcellulose, l'éthylcellulose, etc
- Exsudats et extraits de plantes, également appelés gommes végétales ou gommes naturelles, y compris, mais sans s'y limiter, la gomme arabique (ou la gomme d'acacia), la gomme tragacanthe, la gomme de guar, la gomme de caroube, la gomme karaya, la gomme mesquite, les galactomannanes, la pectine, le polysaccharide de soja soluble
- Extraits marins tels que carraghénane et alginate
- Polysaccharides microbiens et animaux tels que le gellane, le dextrane, le xanthane, le chitosane.

Les polysaccharides peuvent être classés en fonction de leur solubilité dans l'eau. En particulier, la cellulose n'est pas soluble dans l'eau. Selon l'invention, les polysaccharides présentent la capacité d'être solubles dans l'eau.

Les polysaccharides employés dans la formulation de compositions plastiques sont bien connus de l'homme du métier. Ils sont en particulier choisis parmi les dérivés d'amidon comme l'amylose, l'amylopectine, les maltodextrines, le sirop de glucose, les dextrines et les cyclodextrines, les gommes naturelles comme la gomme arabique, la gomme tragacanthe, la gomme de guar, la gomme locust beam, la gomme de karaya, la gomme de mesquite, les galactomannanes, la pectine ou les polysaccharides solubles de soja, les extraits marins comme les carraghénanes et les alginates, et des polysaccharides microbiens ou animaux comme les gellanes, les dextranes, les xanthanes ou le chitosane, et leurs mélanges.

Le polysaccharide peut aussi être un mélange de plusieurs polysaccharides cités ci-dessus. Dans un mode de réalisation préféré, le polysaccharide utilisé est une gomme naturelle, et plus particulièrement la gomme arabique.

Les enzymes utilisées sont des enzymes ayant une activité de dégradation des polyesters. Leur incorporation au sein des produits de matière plastique biodégradables à base de polyesters permet ainsi d'améliorer la biodégradabilité de ces derniers.

Des exemples d'enzymes ayant une activité de dégradation de polyester sont bien connus de l'homme du métier, notamment les dépolymérases, les estérases, les lipases, les cutinases, les carboxylestérases, les protéases ou les polyestérases.

On citera en particulier des enzymes capables de dégrader les polyesters de manière à améliorer la biodégradabilité des articles préparés avec le mélange maître selon l'invention. Dans un mode particulier de l'invention les enzymes sont capables de dégrader le PLA. De telles enzymes et leur mode d'incorporation dans les articles thermoplastiques sont connus de l'homme du métier, notamment décrits dans les demandes de brevets WO 2013/093355, WO 2016/198652, WO 2016/198650, WO 2016/146540 et WO 2016/062695.

Les enzymes utilisées dans le contexte de l'invention sont notamment choisies parmi les protéases et les protéases à sérines. Des exemples de protéases à sérines sont la Proteinase K de *Tritirachium album,* ou des enzymes dégradant le PLA issues *d'Amycolatopsis sp., Actinomadura keratinilytica, Laceyella sacchari LP175, Thermus sp.,* ou Bacillus licheniformis ou d'enzymes commerciales reformulées et connues pour dégrader le PLA telles que Savinase^{®}, Esperase^{®}, Everlase^{®} ou n'importe quelle enzyme de la famille des subtilisines CAS [9014-01-1] ou tout variant fonctionnel.

Les enzymes peuvent être utilisées sous leur forme pure ou enrichie, et éventuellement en tant que mélange avec un ou plusieurs excipient(s).

En particulier, les enzymes sont choisies pour être capables de dégrader au moins un polymère d'un article de matière plastique qui sera obtenu par l'utilisation du mélange maître dans son procédé de fabrication.

Les enzymes sont employées dans le procédé selon l'invention sous la forme d'une formulation liquide. Une formulation liquide selon l'invention est une solution enzymatique et/ou une suspension d'enzymes dans un solvant, notamment une suspension épaisse, capable de s'écouler à température ambiante. La formulation liquide doit être sous une forme adaptée pour être introduite dans le mélangeur par tout moyen usuel d'introduction d'une formule liquide dans un mélangeur. La formulation peut ainsi être introduite via un injecteur ou une pompe péristaltique. L'homme du métier saura déterminer quel dispositif est le plus approprié pour l'ajout de la formulation. Dans un mode de réalisation préféré, la formulation liquide est introduite via une pompe péristaltique. Le solvant est un solvant qui ne dégrade pas les enzymes, et plus particulièrement de l'eau. Selon un mode de réalisation de l'invention, la formulation liquide est essentiellement constituée d'enzymes et du solvant, en particulier de l'eau.

La forme de la formulation dépendra en particulier de la teneur en enzymes. Il est compris que si la teneur en enzymes dépasse le seuil de solubilité de ces dernières dans le solvant, la formulation comprendra des enzymes en suspension et aura l'apparence d'une composition épaisse, mais néanmoins capable de s'écouler. Selon un mode avantageux de réalisation de l'invention, la formulation enzymatique liquide est une solution enzymatique.

Selon un mode de réalisation, la formulation enzymatique liquide comprend de 0,01 à 70% en poids d'enzymes, en particulier de 0,3 à 60% d'enzymes, plus particulièrement de 0,5 à 35% d'enzymes. Notamment, la formulation enzymatique, en particulier la solution enzymatique, peut comprendre en poids une teneur en enzymes de 1%, 5%, 10%, 15%, 20%, 25%, 30%, 35% ou plus.

Le polymère support est un polymère à bas point de fusion et un polymère qui présente avantageusement une température de fusion inférieure à 140°C et/ou une température de transition vitreuse inférieure à 70°C. Il doit également être compatible avec le ou les polymère(s) avec le(s)quel(s) le mélange maître sera mélangé pour la préparation d'articles de matière plastique enzymés.

De tels polymères supports sont bien connus de l'homme du métier. Il s'agit en particulier de polycaprolactone (PCL), polybutylene succinate (PBS), polybutylene succinate adipate (PBSA), polybutylene adipate terephthalate (PBAT), polydioxanone (PDS), polyhdroxyalkanoate (PHA), d'acide polylactique (PLA), ou de leurs copolymères. Il peut aussi s'agir d'un polymère naturel comme l'amidon ou encore un polymère que l'on qualifiera d'universel, c'est à dire compatible avec une large gamme de polymères comme un copolymère de type EVA.

De manière avantageuse, le polymère support a une température de fusion inférieure à 120°C et/ou une température de transition vitreuse inférieure à 30°C.

Le polymère support est généralement un seul polymère tel que défini ci-dessus. Il peut aussi être constitué d'un mélange de ces polymères supports.

Selon un mode particulier de réalisation de l'invention, le polymère support est du PCL.

Selon un mode particulier de réalisation de l'invention, les enzymes du mélange maître ne sont pas capables de dégrader le polymère support.

L'étape a) correspond à l'addition du polysaccharide, des enzymes en formulation liquide et du polymère support dans le mélangeur. Le polysaccharide est sous forme pulvérulente et est introduit dans le mélangeur via un doseur spécifique aux poudres. Les enzymes en formulation liquide sont ajoutées par tout moyen usuel d'introduction d'une solution dans un mélangeur ou tout autre moyen industriel comme une pompe péristaltique ou un injecteur. Dans un mode de réalisation, les enzymes en formulation liquide sont ajoutées via une pompe péristaltique. Le polymère support est sous forme de granulés et est introduit dans le mélangeur via un doseur spécifique aux granulés.

Selon un mode particulier de réalisation, le mélange maître est préparé en mélangeant :
- 60 à 90% de polymère support, en particulier de PCL,
- 10 à 20% de formulation enzymatique liquide, en particulier de solution enzymatique,
- 2 à 15% de polysaccharide, en particulier de gomme arabique.

Dans un mode de réalisation de l'invention, la composition du mélange maître comprend au plus 5% d'enzymes ayant une activité de dégradation des polyesters. La teneur en formulation enzymatique liquide, en particulier solution enzymatique, entrant dans la fabrication du mélange maître dépendra donc de la teneur en enzymes dans sa formulation liquide.

De manière avantageuse, le ratio formulation enzymatique liquide / polysaccharide est calculé de manière à avoir une masse sèche (enzymes et polysaccharide) de 30 à 70% du mélange des deux.

Le ratio polysaccharide/solution enzymatique est déterminé de manière à avoir une masse sèche d'au moins 30% et au plus 55% voire au plus 70%.

L'homme du métier saura adapter les caractéristiques du procédé (température et temps) nécessaires à la réalisation de l'étape a) en fonction des composants (polysaccharide, enzymes et polymère support) utilisés.

Le mélange de l'étape b) se fait à une température à laquelle le polymère support est partiellement ou totalement fondu. Il est entendu que la température de l'étape b) sera déterminée par l'homme du métier de manière à ce qu'elle n'altère pas les enzymes, et plus particulièrement ne diminue pas de manière substantielle leur activité enzymatique de dégradation du polymère support. Selon un mode particulier, la température de l'étape b) est une température inférieure ou égale à la température de fusion (Tm) du polymère support. L'homme du métier pourra choisir le polymère support en fonction de sa température de fusion et de la capacité des enzymes choisis à résister à cette température dans le procédé selon l'invention. Dans un mode particulier, la température de l'étape b) est comprise entre la température de transition vitreuse (Tg) et la température de fusion (Tm) du polymère support. De manière alternative, la température de l'étape b) est fixée à une température égale ou au-dessus de la Tm du polymère support.

De manière générale, la température de l'étape b) peut être comprise entre 40 et 200°C. dans un mode de réalisation, la température est supérieure à 40°C, voire supérieure à 50°C. La température est préférentiellement comprise entre 55 et 175°C. Dans un mode de réalisation préféré, la température de l'étape b) est ajustée selon la nature du polymère utilisé. Ainsi, la température de l'étape b) se situe ou correspond généralement à la température de fusion du polymère utilisée. Typiquement, la température ne dépasse pas 300°C, plus particulièrement, la température ne dépasse pas 250°C.

On cherchera à maintenir une température du mélange à l'étape b) qui soit la plus basse possible tout en permettant un mélange et une dispersion homogène des enzymes et du polysaccharide dans le polymère support.

Dans un mode particulier, le polymère support est le PCL et la température de l'étape b) de mélange est d'environ 60°C, de 55 à 65°C.

Le mélange des composants polysaccharide, enzymes et polymère support à l'étape b), est effectué pendant une durée de 10 à 35 secondes. Dans un mode particulier, le mélange dure entre 15 et 35 secondes, en particulier environ 20 secondes, environ 25 secondes ou environ 30 secondes.

Au cours du processus de réalisation du mélange maître, la température est graduellement augmentée afin d'assurer un mélange homogène et constant tout en préservant au mieux les caractéristiques et propriétés de chacun des composants.

De manière avantageuse, le temps de résidence de la composition polysaccharide/enzymes dans le polymère à une température au-dessus de 100°C au sein du mélangeur (étape b)) est aussi court que possible. Il est préférentiellement compris entre 5 secondes et 10 minutes. Cependant un temps de résidence inférieur à 5 minutes est préféré. Dans un mode de réalisation préféré, celui-ci est inférieur à 3 minutes, et éventuellement inférieur à 2 minutes.

Le mélange maître obtenu à l'étape c) est sous forme solide. Il est avantageusement récupéré sous forme de granulés. Ces granulés pourront être stockés, transportés, et incorporés dans la fabrication de produits ou articles en matière plastique, quel que soit leur forme et leur utilisation, que l'on peut appeler « produits finaux ». Il peut s'agir de films, ou de pièces souples ou solides de formes et volumes adaptés à leurs usages.

La formulation du mélange maître peut comprendre une charge minérale. Dans ce cas, le composé minéral est introduit lors de l'étape a), avec l'addition du polysaccharide de la formulation enzymatique liquide et du polymère support dans le mélangeur.

Plusieurs minéraux peuvent être utilisés. Des exemples sont la calcite, les sels de carbonate ou les métaux carbonates tels que le carbonate de calcium, le carbonate de potassium, le carbonate de magnésium, le carbonate d'aluminium, le carbonate de zinc, le carbonate de cuivre, la craie, la dolomite ; les sels de silicate, tels que le silicate de calcium, le silicate de potassium, le silicate de magnésium, le silicate d'aluminium, ou un mélange de ceux-ci, comme les micas, les smectites comme la montmorillonite, la vermiculite, et sépiolite-palygorskite ; les sels de sulfates, tels que le sulfate de barium ou le sulfate de calcium (gypsum), le mica ; les sels d'hydroxyde ou les métaux d'hydroxyde comme l'hydroxyde de calcium, l'hydroxyde de potassium (potasse), l'hydroxyde de magnésium, l'hydroxyde d'aluminium, l'hydroxyde de sodium (soude caustique), l'hydrotalcite ; les métaux oxydes ou les sels d'oxyde comme l'oxyde de magnésium, l'oxyde de calcium, l'oxyde d'aluminium, l'oxyde de fer, l'oxyde de cuivre, l'argile, l'asbestis, la silice, le graphite, le noir de carbone ; les fibres de métal ou les pétales de métal ; les fibres de verre ; les fibres magnétiques ; les fibres céramiques et des dérivés et/ou des mélanges de ceux-ci.

Dans un mode de réalisation préféré, la charge minérale utilisée est du carbonate de calcium. De manière générale, le mélange maître est formulé avec :
- 60 à 90% de polymère support, en particulier PCL,
- 10 à 20% de formulation enzymatique liquide, en particulier solution enzymatique,
- 2 à 15% de polysaccharide, en particulier gomme arabique et
- 0 à 20% de charge minérale, en particulier carbonate de calcium.

Le mélange maître peut aussi comprendre la présence d'un ou plusieurs composés. En particulier, le mélange maître peut comprendre un ou plusieurs additifs. De manière générale, les additifs sont utilisés afin d'améliorer des propriétés spécifiques du produit final. Par exemples, les additifs peuvent être choisis parmi les plastifiants, les agents de coloration, les auxiliaires technologiques, les agents rhéologiques, les agents antistatiques, les agents anti-UV, les agents de renforcement, les agents de compatibilité, les agents de retardement de flamme, les antioxydants, les pro-oxydants, les stabilisateurs de lumière, les pièges à oxygène, les adhésifs, les produits, les excipients, etc.

De manière avantageuse, le mélange maître comprend moins de 20% en poids d'additifs et préférentiellement moins de 10% par rapport au poids total du mélange maître. En général, la composition du mélange maître comprend de 0% à 10% en poids d'additifs par rapport au poids total du mélange maître.

La composition du mélange maître après formulation comprend entre 5% et 30% en poids de formulation enzymatique liquide telle que définie plus haut, par rapport au poids total du mélange maître. Dans un mode de réalisation, la formulation enzymatique liquide représente entre 8% et 22% en poids par rapport au poids total de la composition. Dans un mode préféré, le mélange maître comprend entre 10% et 20% de formulation enzymatique liquide, en poids de sa composition.

Le procédé de fabrication du mélange maître est réalisé dans un mélangeur. L'homme du métier connait différents types de mélangeurs susceptibles d'être employés pour la fabrication de ces mélanges maîtres de polymères.

Dans un mode préféré de réalisation, le mélangeur est une extrudeuse. Celle-ci peut être de type monovis ou bivis. Elle est préférentiellement de type bivis.

En particulier, le procédé est mis en œuvre dans une extrudeuse comprenant au moins 3 zones, une zone de tête où sont introduits les premiers composants, une zone de mélange et une zone de sortie par laquelle le mélange maître est récupéré, avec les étapes a) à c) suivantes :
a) l'introduction séparée et simultanée en zone de tête d'un polysaccharide, d'une formulation enzymatique liquide et d'un polymère support, et le cas échéant d'une charge minérale, tels que définis ci-dessus,
b) le mélange des composants dans la zone de mélange à une température à laquelle le polymère support est partiellement ou totalement fondu,
c) la récupération du mélange maître en sortie de l'extrudeuse.

La personne du métier saura adapter les caractéristiques de l'extrudeuse (i.e. la longueur et le diamètre de la/des vis, les éléments de vis, les zones de dégazages...) et le temps de résidence du polysaccharide, des enzymes et du polymère support en fonction des contraintes de temps et de température des différentes étapes du procédé de l'invention.

En particulier, la température en zone de tête est inférieure à la température de fusion du polymère support et la température de la zone de mélange est supérieure à la température de la zone de tête, notamment telle que définie plus haut pour les températures de mélange.

La zone de mélange peut elle-même comprendre plusieurs zones et l'homme du métier pourra, si besoin, adapter les températures de chaque zone notamment en fonction des enzymes et des polymères supports employés.

Le mélange maître peut être obtenu sous forme de granulés préparés selon les techniques usuelles. Ces granulés pourront être stockés, transportés et utilisés dans la fabrication d'articles de matière plastique biodégradables, que l'on peut appeler « articles finaux ».

Lorsqu'il est sous forme de granulés, le mélange maître peut être séché pour son stockage. Les méthodes de séchage sont des méthodes usuelles connues de l'homme du métier, notamment avec l'utilisation d'étuves sous air chaud, d'étuves sous vide, de dessiccateurs, de micro-ondes ou de lit fluidisé. La température de séchage et sa durée dépendront d'une part de la teneur en eau apportée par la solution enzymatique dans la préparation du mélange maître, mais aussi des températures de fusion et de transition vitreuse du polymère support employé.

Une fois sec, la composition du mélange maître comprend avantageusement de :
- 60% à 95% de polymère support,
- 0,5% à 7% d'enzyme,
- 2% à 27% de polysaccharide, et
- 0% à 30% de charge minérale.

Le taux d'humidité est généralement de 0,5% ou moins et de préférence inférieur à 0,3%.

Le mélange maître obtenu sous forme de granulés, peut ensuite entrer dans la fabrication de produits de matière plastique biodégradables ou « articles finaux ». Il peut s'agir de films, ou de pièces souples ou solides de formes et volumes adaptés à leurs usages.

L'article de matière plastique biodégradable est obtenu par mélange du mélange maître comprenant les enzymes avec au moins un polymère susceptible d'être dégradé par lesdites enzymes.

L'invention concerne donc un procédé de préparation d'un article en matière plastique ou un pré-mélange tels que définis ci-dessous comprenant un polymère susceptible d'être dégradé par des enzymes et des enzymes capables de dégrader ledit polymère, ledit procédé comprenant les étapes de préparation d'un mélange maître comprenant des enzymes capables de dégrader ledit polymère, un polysaccharide, et un polymère support, et éventuellement une charge minérale, le mélange maître étant préparé dans un mélangeur par un procédé comprenant les étapes suivantes de :
a) introduction séparée et simultanée dans le mélangeur des enzymes en formulation liquide, du polysaccharide et du polymère support, et le cas échéant d'une charge minérale, tels que définis ci-dessus
b) mélange des composants, et
c) récupération du mélange maître,
puis mélange dudit polymère susceptible d'être dégradé par des enzymes avec le mélange maître.

De manière avantageuse, ledit polymère susceptible d'être dégradé par les enzymes est un polyester biodégradable. Ces polyesters sont bien connus de l'homme du métier, comme l'acide polylactique (PLA), l'acide polyglycolique (PGA), le polyhydroxyalkanoate (PHA), le polycaprolactone (PCL), le polybutylene succinate (PBS), le polybutylene succinate adipase (PBSA), le polybutylene adipate terephtalate (PBAT), l'amidon plastifié et les mélanges de ceux-ci.

Ces polyesters sont choisis pour leurs propriétés physico-chimiques en fonction de l'article final et des propriétés qui seront recherchées, en particulier ses propriétés mécaniques mais aussi leur couleur ou leur transparence.

Les polyesters biodégradables employés pour la préparation des articles finaux ont des propriétés physicochimiques identiques ou différentes des polyesters employés comme polymères supports dans le mélange maître selon l'invention.

Dans un mode de réalisation préféré, le polyester pouvant être dégradé par les enzymes comprend du PLA, seul ou en mélange avec un autre polyester ci-dessus, en particulier un mélange PLA/PBAT.

L'article de matière plastique biodégradable est ainsi constitué du mélange maître et d'un polymère biodégradable.

La composition de l'article de matière plastique biodégradable comprend en plus du polymère biodégradable de 0,5% à 20% de mélange maître enzymé.

Les méthodes de préparation de ces articles finaux sont bien connues de l'homme du métier, comprenant en particulier les techniques usuelles de la plasturgie telles que l'extrusion-gonflage, l'extrusion-soufflage, l'extrusion de film coulé, le calandrage et le thermoformage, le moulage par injection, le moulage par compression, le rotomoulage, le revêtement, la stratification, l'expansion, la pultrusion, la compression-granulation. De telles opérations sont bien connues de l'homme du métier, qui adaptera facilement les conditions du procédé en fonction du type d'articles en matière plastique prévu (par exemple température, temps de séjour, etc.).

Pour la préparation des articles de matière plastique biodégradables, on peut mélanger le mélange maître avec les autres constituants de la composition pour leur mise en forme. On peut également préparer un pré-mélange ou « compound » comprenant le mélange maître et au moins le polymère biodégradable. Ce pré-mélange sous forme solide, en particulier sous forme de granulés, peut être stocké puis transporté avant d'être employé pour la mise en forme de l'article final, seul ou associé à d'autres constituants selon la composition finale de l'article final.

Avantageusement, le pré-mélange comprend :
- de 8% à 99% en poids de polymère biodégradable, préférentiellement du PLA,
- de 0,01% à 5% en poids d'un polysaccharide, préférentiellement une gomme naturelle telle que la gomme arabique
- de 0,1% à 20% en poids d'un polymère support, tel que défini ci-dessus, en particulier du PCL, et
- de 0,01% à 2% en poids d'enzymes ayant une activité de dégradation de polymère biodégradable, plus particulièrement ayant une activité de dégradation de PLA, et le cas échéant
- de 0 à 35% en poids de charge minérale.

Les articles finaux peuvent être des films, de pièces souples ou solides de formes et volumes adaptés à leurs usages. Des exemples d'articles de matière plastique biodégradables concernés par l'invention sont les films, les films de paillage, les films de routage, les films alimentaires ou non alimentaires ; les emballages tels que les blisters d'emballage, les barquettes ; la vaisselle jetable comme les gobelets, les assiettes ou encore les couverts ; les bouchons et les couvercles ; les capsules de boisson ; et les articles horticoles.

De manière avantageuse, la composition de l'article de matière plastique est la suivante :
- 60% à 98% en poids de polymère ou mélange de polymères biodégradable(s),
- 0,01% à 5% en poids d'un polysaccharide, préférentiellement une gomme naturelle telle que la gomme arabique,
- 0,01% à 20% en poids d'un polymère support, tel que défini ci-dessus,
- 0,01% à 2% en poids d'enzymes ayant une activité de dégradation de polymère biodégradable,
- 0% à 35% en poids de charge minérale,
- 0% à 5% en poids d'additifs.

Les articles de matière plastique biodégradables obtenus avec le mélange maître enzymé peuvent être souples et/ou rigides.

Dans le cas des articles souples, le polyester pouvant être dégradé par les enzymes comprend du PLA. Dans un mode de réalisation ; le polyester biodégradable est un mélange PBAT/PLA dont le rapport pondéral va préférentiellement de 10/90 à 20/80, plus préférentiellement de 13/87 à 15/85.

Dans un autre mode de réalisation, le polyester biodégradable est un mélange PBAT/PLA dont le rapport pondéral va de 10/90 à 30/70, de 10/90 à 40/60, de 10/90 à 50/50, de 10/90 à 60/40, de 10/90 à 70/30, de 10/90 à 80/20, de 10/90 à 90/10.

Dans un autre mode de réalisation, le polyester biodégradable est un mélange PBAT/PLA dont le rapport pondéral est inférieur à 10/90, égal ou inférieur à 9/91, égal ou inférieur à 8/92, égal ou inférieur à 7/93, égal ou inférieur à 6/94, égal ou inférieur à 5/95, égal ou inférieur à 4/96, égal ou inférieur à 3/97, égal ou inférieur à 2/98, égal ou inférieur à 1/99.

Dans un autre mode de réalisation, le polyester biodégradable est du PLA.

Les articles de matière plastique biodégradables souples sont caractérisés par une épaisseur inférieure à 250 µm, préférentiellement par une épaisseur inférieure à 200 µm. Dans un mode de réalisation préféré, les films ont une épaisseur inférieure à 100 µm, plus avantageusement inférieure à 50 µm, 40 µm ou 30 µm, préférentiellement entre 10 et 20 µm. Plus préférentiellement, l'épaisseur de l'article souple est de 15 µm. Des exemples sont les films, tels que les films alimentaires, les films de routage, films industriels ou les films de paillages et les sacs.

Avantageusement, la composition de l'article souple comprend :
- de 70% à 98% en poids de polymère ou mélange de polymères biodégradable(s),
- de 0,01% à 5% en poids d'un polysaccharide, préférentiellement une gomme naturelle telle que la gomme arabique
- de 0,1% à 20% en poids d'un polymère support, tel que défini ci-dessus, et
- de 0,01% à 2% en poids d'enzymes ayant une activité de dégradation de polymère biodégradable,
- de 0% à 5% en poids de charge minérale, notamment de 0,01% à 5% en poids, en particulier de 0,05 à 5% en poids,
- de 0% à 5% en poids d'additifs.

La composition selon l'invention est particulièrement adaptée pour la réalisation de films plastiques. Les films selon l'invention peuvent être produits selon les méthodes usuelles de la technique, en particulier par extrusion-gonflage. Les films peuvent être préparés à partir de granulés de composition selon l'invention qui sont fondus selon les techniques usuelles, en particulier par extrusion.

Les films de composition telle que définie précédemment avec des enzymes peuvent être des films monocouches ou multicouches. Dans le cas d'un film multicouche, au moins une des couches est de composition telle que définie précédemment. Les films monocouches et multicouches, de composition telles que définies précédemment, ont à la fois une forte teneur en PLA et conservent des propriétés mécaniques telles que recherchées pour la préparation de films biodégradables et biosourcés, notamment pour l'emballage de produits alimentaires et non alimentaires. A cet effet, les constituants de la composition selon l'invention seront préférentiellement choisis parmi les produits compatibles avec un usage alimentaire.

Le film multicouche peut être un film comprenant au moins 3 couches, de type ABA, ABCA ou ACBCA, les couches A, B et C étant de compositions différentes. Dans un mode préféré, les films multicouches sont de type ABA ou ACBCA.

Généralement les couches A et B comprennent du PLA et/ou un polyester, avantageusement d'une composition selon l'invention. Les couches C, si présentes, sont là pour apporter des propriétés particulières aux articles selon l'invention, plus particulièrement pour apporter des propriétés barrières aux gaz et notamment à l'oxygène. De tels matériaux barrières sont bien connus de l'homme du métier, et notamment le PVOH (alcool polyvinylique), le PVCD (polychlorure de vinyle), le PGA (acide polyglycolique), la cellulose et ses dérivés, les protéines de lait, ou des polysaccharides et leurs mélanges en toutes proportions.

Dans le cas de films multicouches tels que définis plus haut, et en particulier de films de type ABA, ABCA ou ACBCA, les enzymes peuvent être présentes dans toutes les couches ou bien dans une seule des couches, par exemple dans les couches A et B ou seulement dans la couche A ou dans la couche B.

Selon un mode particulier de réalisation de l'invention, les deux couches A sont constituées d'une composition selon l'invention comprenant le PLA, le polyester et du poly propylene glycol diglycidyl ether (PPGDGE), sans enzymes. Les enzymes sont dans la couche B, soit dans une composition selon l'invention avec enzymes telle que définie plus haut, soit dans une composition particulière en particulier une composition d'enzyme dans un polymère à bas point de fusion définie plus haut.

Selon les modes de réalisation, la composition de la couche enzymée des articles souples (mono- ou multicouche) peut comprendre jusqu'à 95% en poids de polymère biodégradable, préférentiellement du PLA. Ainsi, la couche enzymée peut comprendre de 8% à 50%, de 8% à 60%, de 8% à 70%, de 8% à 80% ou encore de 8% à 90% en poids de polymère biodégradable. Avantageusement, la composition de la couche enzymée des articles souples (mono- ou multicouche) comprend :
- de 8% à 95% en poids de polymère biodégradable, préférentiellement du PLA, notamment de 8% à 70%, de 8% à 60%, de 8% à 50%, ou de 8% à 40%,
- de 0,02% à 4% en poids d'un polysaccharide, préférentiellement une gomme naturelle telle que la gomme arabique
- de 0,1% à 19% en poids d'un polymère support, tel que défini ci-dessus, et
- de 0,05% à 2% en poids d'enzymes ayant une activité de dégradation de polymère biodégradable, plus particulièrement ayant une activité de dégradation de PLA, et le cas échéant
- de 0 à 5% en poids de charge minérale, notamment de 0,01% à 5% en poids, en particulier de 0,05% à 5% en poids.

Concernant les articles rigides, le polyester biodégradable est du PLA, préférentiellement un mélange PLA/carbonate de calcium dont le rapport pondéral va de 100/0 à 25/75, préférentiellement de 95/5 à 45/55, plus préférentiellement de 90/10 à 50/50. Dans un autre mode de réalisation, le polyester biodégradable est un mélange PBAT/PLA dont le rapport pondéral va préférentiellement de 10/90 à 80/20, plus préférentiellement de 20/80 à 60/40.

Les articles rigides possèdent une épaisseur comprise entre 200 µm et 5 mm, entre 150 µm et 5 mm, préférentiellement entre 200 µm et 3 mm, ou entre 150 µm et 3 mm. Dans un mode de réalisation, les articles ont une épaisseur comprise entre 200 µm et 1 mm, entre 150 µm et 1 mm, préférentiellement entre 200 µm et 750 µm ou entre 150 µm et 750 µm. Dans un autre mode de réalisation, l'épaisseur est de 450 µm.

Des exemples de tels articles de matière plastique biodégradables sont les gobelets, les assiettes, les couverts, les barquettes, les capsules de boisson et les blisters d'emballage, de manière plus générale des emballages alimentaires ou cosmétiques, ou des produits horticoles. Avantageusement, la composition de l'article rigide comprend :
- de 60% à 95% en poids de polymère ou mélange de polymères biodégradable(s),
- de 0,01% à 5% en poids d'un polysaccharide, préférentiellement une gomme naturelle telle que la gomme arabique
- de 0,1% à 20% en poids d'un polymère support, tel que défini ci-dessus,
- de 0,01% à 2% en poids d'enzymes ayant une activité de dégradation de polymère biodégradable,
- de 0% à 35% en poids de charge minérale, notamment 0,01 à 35% en poids,
- de 0% à 5% en poids d'additifs.

Dans un autre mode de réalisation, la composition de l'article rigide comprend :
- de 60% à 80% en poids de polymère ou mélange de polymères biodégradable(s),
- de 0,01% à 5% en poids d'un polysaccharide, préférentiellement une gomme naturelle telle que la gomme arabique
- de 0,1% à 20% en poids d'un polymère support, tel que défini ci-dessus, et
- de 0,01% à 2% en poids d'enzymes ayant une activité de dégradation de polymère biodégradable,
- de 8% à 35% en poids de charge minérale,
- de 0% à 5% en poids d'additifs.

La composition de l'article rigide comprend ainsi plus de 60 % en poids de polymère ou mélange de polymère(s) biodégradables, voire plus de 70%, voire plus de 80%, voire plus de 90%.

La teneur de la charge minérale dans l'article rigide est comprise entre 0,01% et 35% en poids selon la nature de la charge minérale.

Selon des modes de réalisation, l'article rigide comprend ainsi plus de 0,01%, plus de 0,1%, plus de 1%, voire plus de 2%, voire plus de 3% en poids de charge minérale.

Dans d'autre modes de réalisation, la quantité en poids de charge minérale est supérieure ou égale à 4%, supérieure ou égale à 5%, supérieure ou égale à 6%, supérieure ou égale à 7%, ou supérieure ou égale à 8%.

Encore dans d'autres modes de réalisation, la charge minérale comprise dans l'article rigide est de 10 à 35% en poids, de 15% à 30%, ou de 20% à 28% en poids.

Qu'ils soient souples ou rigides, les articles finaux peuvent également comprendre des plastifiants, des compatibilisants et autres additifs usuels entrant dans la composition de matières plastiques, comme des pigments ou des colorants, des agents démoulants, des modificateurs d'impact, agent antiblock etc.

Des exemples de plastifiants sont les esters de citrate et les oligomères d'acide lactique (OLA). Les esters de citrate sont des plastifiants connus de l'homme du métier, en particulier comme matériaux biosourcés. On citera notamment le triéthyl citrate (TEC), le triéthyl acétyl citrate (TEAC), le tributyl citrate (TBC), le tributyl acétyl citrate (TBAC). De manière préférentielle, l'ester de citrate employé comme plastifiant dans la composition selon l'invention est le TBAC.

Les OLAs sont également des plastifiants connus de l'homme du métier, en particulier comme matériaux biosourcés. Il s'agit d'oligomères d'acide lactique de poids moléculaire inférieur à 1500 g/mol. Ils sont de préférence des esters d'oligomères d'acides lactiques, leur terminaison acide carboxylique étant bloquée par estérification avec un alcool, en particulier un alcool linéaire ou ramifié en C1-C10, avantageusement un alcool en C6-C10, ou un mélange de ces derniers. On citera notamment les OLAs décrits dans la demande de brevet EP 2 256 149 avec leur mode de préparation, et les OLAs commercialisés par la société Condensia Quimica sous la marque Glyplast^{®}, en particulier les références Glyplast^{®} OLA 2, qui a un poids moléculaire de 500 à 600 g/mol et Glyplast^{®} OLA 8 qui a un poids moléculaire de 1000 à 1100 g/mol. Selon un mode préféré de réalisation de l'invention, les OLAs ont un poids moléculaire d'au moins 900 g/mol, de préférence de 1000 à 1400 g/mol, plus préférentiellement de 1000 à 1100 g/mol

Les poly (propylene glycol) diglycidyl ether sont également appelés éthers de glycidyls, décrits notamment comme « plastifiants réactifs » dans la demande de brevet WO 2013/104743, employés pour la préparation de copolymères blocs avec du PLA et du PBAT. Ils sont également identifiés comme résine époxy liquide, de la société DOW, commercialisée sous la référence « D.E.R.^{™} 732P », ou encore comme résine époxy aliphatique, de la société HEXION, commercialisée sous la référence « Epikote^{™} Resin 877 ».

La composition selon l'invention peut comprendre de manière optionnelle d'autres compatibilisants PLA/Polyesters associés au PPGDGE. De tels compatibilisants PLA/Polyesters sont bien connus de l'homme du métier, notamment choisis parmi les polyacrylates, les terpolymères d'éthylène, d'ester acrylique et de méthacrylate de glycidyle (par exemple commercialisé sous la marque Lotader^{®} par la société Arkema), les copolymères triblocs PLA-PBAT-PLA, les PLA greffés d'anhydride maléique (PLA-g-AM) ou les PBAT greffés d'anhydride maléique (PBAT-g-AM), en particulier du poly(éthylène-co-méthyl acrylate-co-glycidyl méthacrylate) décrits notamment par Dong & al. (International Journal of Molecular Sciences, 2013, 14, 20189-20203) et Ojijo & al. (Polymer 2015, 80, 1-17), plus particulièrement commercialisés sous la dénomination JONCRYL^{®} par la société BASF, préférentiellement le grade ADR 4468.

### EXEMPLES

### Exemple 1 : Utilisation du mélange maitre dans les articles souples

### I. Préparation d'un mélange de polymère support et d'enzymes

### 1. Préparation d'un mélange-maître dans des conditions de l'état de la technique

Le mélange **A1** de polymère support et d'enzymes est préparé à partir de granulés de polycaprolactone (PCL), d'un polysaccharide (gomme arabique), d'une charge minérale (carbonate de calcium, CaCO₃) et d'enzymes en solution. Le mélange de polymère support et d'enzymes a été fabriqué avec une bi-vis co-rotative Clextral Evolum 25 HT comprenant 11 zones pour lesquelles la température est indépendamment contrôlée et régulée. Les enzymes en solution, la gomme arabique et le carbonate de calcium ont été introduites simultanément au début de l'extrudeuse afin de réaliser le mélange selon un profil de température croissant compris entre 25 et 50°C. Les enzymes en solution sont introduites à 2,6kg/h à l'aide d'une pompe péristaltique. La gomme arabique est, quant à elle, introduite à 1,4kg/h à l'aide d'un doseur spécifique aux poudres et le carbonate de calcium est introduit à 2kg/h. Le PCL, aussi appelé polymère support, est introduit à 14kg/h dans un état partiellement voire totalement fondu entre la zone 5 et la zone 6 de l'extrudeuse à une température de 75°C.

Le mélange **A2** est fait dans les mêmes conditions que le mélange **A1,** à un jour différent.

Le mélange **A3** de polymère support et d'enzymes est préparé à partir de granulés de polycaprolactone (PCL), d'un polysaccharide (gomme arabique), d'une charge minérale (carbonate de calcium, CaCO₃) et d'enzymes en solution. Le mélange de polymère support et d'enzymes a été fabriqué avec une bi-vis co-rotative Clextral Evolum 25 HT comprenant 11 zones pour lesquelles la température est indépendamment contrôlée et régulée. Les enzymes en solution, la gomme arabique et le carbonate de calcium ont été introduites simultanément au début de l'extrudeuse afin de réaliser le mélange selon un profil de température croissant compris entre 25 et 50°C. Les enzymes en solution sont introduites à 2,2kg/h à l'aide d'une pompe péristaltique. La gomme arabique est, quant à elle, introduite à 1,8kg/h à l'aide d'un doseur spécifique aux poudres et le carbonate de calcium est introduit à 2kg/h. Le PCL, aussi appelé polymère support, est introduit à 14kg/h dans un état partiellement voire totalement fondu entre la zone 5 et la zone 6 de l'extrudeuse à une température de 75°C.

La granulation de chaque mélange est faite avec une coupe sous eau. Les granulés sont séchés à 45°C jusqu'à 0,3% d'humidité.

### 2. Préparation d'un mélange-maître selon procédé de l'invention

Le mélange **B1** de polymère support et d'enzymes est préparé à partir de granulés de polycaprolactone (PCL), d'un polysaccharide (gomme arabique) et d'enzymes en solution selon le procédé de l'invention.

Le mélange de polymère support et d'enzymes a été fabriqué avec une extrudeuse bi-vis CLEXTRAL EV25HT comprenant 11 zones pour lesquelles la température est indépendamment contrôlée et régulée selon un profil de température croissant compris entre 25 et 50°C. Le PCL, les enzymes en solution et la gomme arabique sont introduits séparément et simultanément en tête de la bivis. Le PCL est introduit à 16kg/h, les enzymes en solution sont introduites à 2,2kg/h à l'aide d'une pompe péristaltique et la gomme arabique est introduite à 1,8kg/h à l'aide d'un doseur spécifique aux poudres.

Le mélange **B2** est fait dans les mêmes conditions que le mélange **B1,** à un jour différent.

Le mélange **B3** de polymère support et d'enzymes est préparé à partir de granulés de polycaprolactone (PCL), d'un polysaccharide (gomme arabique), d'une charge minérale (carbonate de calcium, CaCO₃) et d'enzymes en solution selon le procédé de l'invention.

Le mélange de polymère support et d'enzymes a été fabriqué avec une extrudeuse bi-vis CLEXTRAL EV25HT comprenant 11 zones pour lesquelles la température est indépendamment contrôlée et régulée selon un profil de température croissant compris entre 25 et 60°C. Le PCL, les enzymes en solution, la gomme arabique et le carbonate de calcium sont introduits séparément et simultanément en tête de la bivis. Le PCL est introduit à 14kg/h, les enzymes en solution sont introduites à 2,2kg/h à l'aide d'une pompe péristaltique, la gomme arabique est introduite à 1,8kg/h à l'aide d'un doseur spécifique aux poudres et le carbonate de calcium est introduit à 2kg/h.

### II. Produits commerciaux

Dans ces exemples, du PCL commercialisé sous la référence Capa^{™} 6500 par la société Perstorp, du carbonate de calcium commercialisé sous la référence OMYAFILM 707-OG par la société Omya, de la gomme arabique commercialisée sous la référence InstantGum AA par la société Nexira ont été utilisés.

Un mix PLA/PBAT commercialisé sous la référence ECOVIO F2223 par la société BASF a été utilisé.

### III. Production des films

Pour l'extrusion gonflage, une ligne de laboratoire Labtech LF-250, laize 20mm, vis de 30 L/D type LBE20-30/C a été utilisée. La vitesse de vis se situe entre 50 et 60 tpm, les vitesses de tirage haut et bas sont situées entre 4,3 et 5,7 m/min.

Les températures d'extrusion gonflage sont détaillées dans les tableaux 1a, 1b et 1c.

**Tableau 1a : Températures d'extrusion gonflage pour les films 1 et 2 avec respectivement les mélanges A1 et A2.**

| Zone | Z1 | Z2 | Z3 | Z4 | Filière #1 | Filière #2 |
|---|---|---|---|---|---|---|
| Température (°C) | 150 | 150 | 150 | 150 | 155 | 150 |

**Tableau 1b : Températures d'extrusion gonflage pour les films 4 et 5 avec respectivement les mélanges B1 et B2.**

| Zone | Z1 | Z2 | Z3 | Z4 | Filière #1 | Filière #2 |
|---|---|---|---|---|---|---|
| Température (°C) | 125 | 165 | 155 | 155 | 160 | 160 |

**Tableau 1c : Températures d'extrusion gonflage pour les films 3 et 6 avec respectivement les mélanges A3 et B3.**

| Zone | Z1 | Z2 | Z3 | Z4 | Filière #1 | Filière #2 |
|---|---|---|---|---|---|---|
| Température (°C) | 135 | 145-150 | 155 | 155 | 160 | 160 |

Les films ont une épaisseur moyenne de 15µm. Les épaisseurs ont été mesurées avec un micromètre électronique Positector.

Ces films sont transparents, sans rugosité et aucun défaut passant n'a été identifié. La bulle a été stable pour toutes les extrusions gonflage. L'ouverture du film après l'extrusion gonflage a été qualifiée de standard, sans difficultés.

### IV. Méthode d'analyses

Les propriétés mécaniques en traction et en déchirure peuvent être mesurées à l'aide d'une machine de type Zwick ou Llyod, équipée d'un capteur de 50 N ou d'un capteur de 5 kN. Les propriétés sont mesurées dans deux directions différentes : dans le sens longitudinal et dans le sens transversal. Les propriétés mécaniques en traction et en déchirure sont mesurées respectivement selon les normes EN ISO 527-3 et ISO 6383-1

Quant à la résistance à la perforation, elle est mesurée à l'aide d'un Dart-Test selon la norme NF EN ISO 7765-1.

L'évaluation de la biodégradabilité des films a été évaluée avec un test de dépolymérisation effectué selon le protocole suivant : 100 mg de chaque échantillon ont été introduit dans un vial plastique contenant 50 mL de solution tampon à pH 8. La dépolymérisation est lancée en incubant chaque échantillon à 45°C, dans un incubateur agité à 150 RPM. Une aliquote de 1mL de solution tampon est prélevée régulièrement et filtrée à l'aide d'une seringue à filtre de 0.2 µm afin d'être analysée par chromatographie liquide à haute performance (HPLC) avec une colonne Aminex HPX-87H pour mesurer la libération d'acide lactique (AL) et son dimère. Le système de chromatographie utilisé est une machine HPLC Nexan Series, SHIMADZU comprenant une pompe, un échantillonneur automatique, une colonne thermostatée à 50°C et un détecteur d'UV à 220 nm. L'éluant est une solution de H₂SO₄ à 5 mM. L'injection est de 20 µL d'échantillon. L'acide lactique est mesuré à partir de courbes standard préparées à partir d'acide lactique commercial.

L'hydrolyse des films plastiques est calculée à partir de l'acide lactique et du dimère d'acide lactique libéré. Le pourcentage de dépolymérisation est calculé en regard du pourcentage de PLA dans l'échantillon.

### V. Résultats d'analyses

### Densité des granulés par pycnométrie

Le mélange-maître **A1** issu du mode de préparation de l'état de la technique décrit dans le paragraphe I.1 a une densité équivalente à celle du mélange-maître **B1** issu du mode de préparation de l'invention décrit dans le paragraphe I.2, à savoir 1,03 g/cm³ en moyenne.

Le mélange-maître **A2** issu du mode de préparation de l'état de la technique décrit dans le paragraphe I.1 a une densité équivalente à celle du mélange-maître **B2** issu du mode de préparation de l'invention décrit dans le paragraphe I.2, à savoir 1,05 g/cm³ en moyenne.

Le mélange-maître **A3** issu du mode de préparation de l'état de la technique décrit dans le paragraphe I.1 a une densité équivalente à celle du mélange-maître **B3** issu du mode de préparation de l'invention décrit dans le paragraphe I.2, à savoir 1,1 g/cm³ en moyenne.

Le mode de préparation du mélange-maître polymère support et enzymes n'a pas d'impact sur la densité du compound final.

### Mesures d'indice de fluidité (MFI)

Le mélange-maître **A1** a un indice de fluidité équivalent à celui du mélange-maître **B1,** à savoir 10-10,5 pour une analyse réalisée à 160°C et 2,16kg.

Le mélange-maître **A3** a un indice de fluidité équivalent à celui du mélange-maître **B3,** à savoir 10-10,5 pour une analyse réalisée à 160°C et 2,16kg.

Le mode de préparation du mélange-maître polymère support et enzymes n'a pas d'impact sur la fluidité du compound final.

### Analyses thermogravimétriques

Les analyses thermogravimétriques réalisées sur ces deux mélanges préparés dans le paragraphe I montrent que tous les composants de la formulation sont retrouvés à des températures de décompositions équivalentes.

**Tableau 2 : Résultats des analyses thermogravimétriques**

| Température de décomposition | Mélange A1 | Mélange B1 |
|---|---|---|
| ~ 305°C | ~ 6,8% | ~ 7,6% |
| ~ 390°C | ~ 74,2% | ~ 86% |
| ~ 450°C | ~ 8,5% | ~ 4% |
| Résidus | ~ 10,6% | ~ 2,9% |

| Température de décomposition | Mélange A2 | Mélange B2 |
|---|---|---|
| ~ 300°C | ~ 7,4% | ~ 7,5% |
| ~ 390°C | ~ 72,4% | ~ 90% |
| ~ 420°C | ~ 9,1% | ~ 0% |
| Résidus | ~ 11,1% | ~ 2,4% |

| Température de décomposition | Mélange A3 1 | Mélange B3 |
|---|---|---|
| ~ 300°C | ~ 7,9% | ~ 8,2% |
| ~ 390°C | ~ 70,6% | ~ 70,4% |
| ~ 450°C | ~ 7,5% | ~ 7% |
| Résidus | ~ 14,1% | ~ 14% |

Les formulations des mélange-maîtres décrites dans le paragraphe I.1 (état de la technique) et I.2 (invention) montrent des différences de compositions entre les compounds **A1** et **B1** ; **A2** et **B2** ainsi que pour **A3** et **B3.** Ces différences sont observées en termes de pertes de masses lors de l'analyse thermogravimétrique.

Seuls les mélanges maîtres **A3** et **B3** sont de mêmes compositions et les pertes de masse ne montrent pas de différences significatives selon le procédé utilisé.

### Composition des films

Les films ont été préparés avec les mélanges polymère support et d'enzymes **A1-A2-A3-B1-B2-B3** préparés en I.1 et I.2 et un grade à base de PLA et PBAT commercialisé sous la référence ECOVIO F2223 par la société BASF et nommé « Compound 1 » dans les exemples ci-dessous. Les compositions de ces différents films sont répertoriées dans le tableau 3.

**Tableau 3 : Récapitulatif des films monocouches réalisés**

| | Composition film | Teneur globale en enzyme (%) |
|---|---|---|
| Film 1 | Compound 1 + Mélange A1 | >0,15% et identique au film 2 |
| Film 2 | Compound 1 + Mélange A2 | >0,15% et identique au film 1 |
| Film 3 | Compound 1 + Mélange A3 | >0,1% |
| Film 4 | Compound 1 + Mélange B1 | > au % d'enzyme des films 1 et 2 et équivalent au film 5 |
| Film 5 | Compound 1 + Mélange B2 | > au % d'enzyme des films 1 et 2 et équivalent au film 4 |
| Film 6 | Compound 1 + Mélange B3 | >0,1% et identique au film 3 |

Le film 1 sert de référence de l'état de la technique pour le film 4 de l'invention.

Le film 2 sert de référence de l'état de la technique pour le film 5 de l'invention.

Le film 3 sert de référence de l'état de la technique pour le film 6 de l'invention.

Le procédé de fabrication du mélange-maître n'a pas d'impact sur le procédé d'extrusion gonflage. Les paramètres de l'extrusion gonflage restent identiques entre le film de la technique et de l'invention. Quel que soit le procédé de fabrication utilisé, l'aspect des films est identique.

### Propriétés mécaniques des films 3 (état de la technique) et 6 (invention)

Les propriétés mécaniques des films 3 et 6 obtenus avec des mélanges-maîtres de compositions identiques ont été mesurées. Les résultats sont présentés dans le tableau 4. Les valeurs indiquées représentent la moyenne de l'ensemble des mesures réalisées.

**Tableau 4 : Caractérisation des propriétés mécaniques des films**

| | | | |
|---|---|---|---|
| Film | | 3 | 6 |
| densité film (g/cm³) film | | 1,23 | 1,23 |
| épaisseur (µm) | | 12 | 11 |

| Propriétés | *Sens de mesure* | | |
|---|---|---|---|
| Contrainte à la rupture (%) | SL | 100% | +0% |
| | ST | 100% | -7% |
| Allongement à la rupture (%) | SL | 100% | -21% |
| | ST | 100% | +47% |
| Module Young (%) | SL | 100% | +24% |
| | ST | 100% | +11% |
| Résistance à la déchirure (%) | ST | 100% | +0% |

| | | | |
|---|---|---|---|
| (avec SL = Sens longitudinal du film et ST = Sens transversal du film) | | | |

Les propriétés mécaniques ainsi mesurées montrent que le film 6 selon l'invention a des propriétés mécaniques maintenues voire supérieures au film 3 selon l'état de la technique.

### Dépolymérisation du PLA des films

Les films 1 et 2 contenant un mélange polymère support et d'enzymes fabriqués dans les conditions classiques, et avec le même taux d'enzymes présentent des résultats de dépolymérisation compris entre 34 et 38% après 20 jours à 28°C. Les films 4 et 5, contenant un mélange polymère support et d'enzymes fabriqués selon le procédé décrit dans l'invention et avec le même taux d'enzymes mais inférieur aux films 1 et 2 présentent des résultats de dépolymérisation entre 28% et 31%.

Les films 3 et 6 dont la teneur en enzymes identique et avec des MB fabriqués par les deux procédés ont un taux de dépolymérisation compris entre 65% et 77% après 5 jours à 45°C.

L'ajout de carbonate de calcium dans la composition du mélange polymère support et d'enzyme favorise la dépolymérisation du PLA. Le procédé de préparation du mélange-maître n'a, quant à lui, pas d'impact négatif sur la performance des enzymes dans le mélange-maître. Le mode de préparation du mélange décrit dans l'invention permet d'atteindre des taux de dépolymérisation proches au procédé classique mais avec moins d'enzymes.

### Exemple 2 : Utilisation du mélange-maître dans les articles souples

### I. Préparation d'un mélange de polymère support et d'enzymes

### 1. Préparation d'un mélange-maître dans des conditions de l'état de la technique (avec une solution enzymatique formulée)

Le mélange **A4** de polymère support et d'enzymes est préparé à partir de granulés de polycaprolactone (PCL) et d'enzymes en solution formulée avec un polysaccharide (gomme arabique). Le mélange **A5** de polymère support et d'enzymes est préparé à partir de granulés de polycaprolactone (PCL), d'une charge minérale (carbonate de calcium, CaCO₃) et d'enzymes en solution formulée avec un polysaccharide (gomme arabique). Les mélanges-maîtres **A4** et **A5** sont préparé selon le mode opératoire de l'exemple 1.I.1 avec un profil de température croissant compris entre 30 et 65°C.

Pour le mélange **A4,** le PCL est introduit en tête de la bivis à 17kg/h, les enzymes en solution formulée avec la gomme arabique sont introduites à 3kg/h à l'aide d'une pompe péristaltique. Pour le mélange **A5,** le PCL est introduit en tête de la bivis à 17kg/h, les enzymes en solution formulée avec la gomme arabique sont introduites à 3kg/h à l'aide d'une pompe péristaltique. Le carbonate de calcium est introduit à 2kg/h à l'aide d'un doseur spécifique aux poudres.

### 2. Préparation d'un mélange-maître selon procédé de l'invention (avec une solution enzymatique non formulée)

Le mélange **B4** de polymère support et d'enzymes est préparé à partir de granulés de polycaprolactone (PCL), d'un polysaccharide (gomme arabique) et d'enzymes en solution.

Le mélange **B5** de polymère support et d'enzymes est préparé à partir de granulés de polycaprolactone (PCL), d'un polysaccharide (gomme arabique), d'une charge minérale (carbonate de calcium, CaCO₃) et d'enzymes en solution.

Les mélanges-maîtres **B4** et **B5** sont préparé selon le mode opératoire de l'exemple 1.I.2 avec un profil de température croissant compris entre 30 et 65°C.

Pour le mélange **B4,** les enzymes en solution sont introduites à 2,2kg/h à l'aide d'une pompe péristaltique, la gomme arabique est introduite à 0,8kg/h à l'aide d'un doseur spécifique aux poudres et le PCL est introduit à 17kg/h en tête de la bivis.

Pour le mélange **B5,** les enzymes en solution sont introduites à 2,2kg/h à l'aide d'une pompe péristaltique, la gomme arabique est introduite à 0,8kg/h à l'aide d'un doseur spécifique aux poudres, le carbonate de calcium est introduit à 2kg/h et le PCL est introduit à 15 kg/h en tête de la bivis.

La granulation de chaque mélange est faite avec une coupe sous eau. Les granulés sont séchés à 45°C jusqu'à 0,3% d'humidité.

### II. Produits commerciaux

Les produits employés pour la préparation des mélanges maîtres et des films sont ceux employés pour l'exemple I.

### III. Production des films

Les films 7 et 8 préparés respectivement avec les mélanges maîtres **A4** (état de la technique) et **B4** (obtenu selon l'invention) sont préparés selon les méthodes décrites pour les films de l'exemple 1.III.

### IV. Méthode d'analyses

Les méthodes d'analyses sont celles décrites pour l'exemple 1.IV.

### V. Résultats d'analyses

### Densité des granulés par pycnométrie

Le mélange-maître **A4** (état de la technique) a une densité équivalente à celle du mélange-maître **B4** obtenu selon l'invention, à savoir 1,06 g/cm³ en moyenne.

Le mélange-maître **A5** (état de la technique) a une densité équivalente à celle du mélange-maître **B5** obtenu selon l'invention, à savoir 1,4 g/cm³ en moyenne.

Le mode de préparation du mélange-maître polymère support et enzymes n'a pas d'impact sur la densité du compound final.

### Mesures d'indice de fluidité (MFI)

Le mélange-maître **A4** a un indice de fluidité équivalent à celui du mélange-maître **B4** issu du mode de préparation de l'invention, à savoir respectivement 15,6 et 14,1 g/10min pour une analyse réalisée à 160 °C et 2,16 kg.

Le mélange-maître **A5** a un indice de fluidité équivalent à celui du mélange-maître **B5** issu du mode de préparation de l'invention, à savoir respectivement 18,3 et 18,9 g/10min pour une analyse réalisée à 160 °C et 2,16 kg.

Le mode de préparation du mélange-maître polymère support et enzymes n'a pas d'impact sur la fluidité du compound final.

### Analyses thermogravimétriques

Les analyses thermogravimétriques réalisées sur les mélanges-maîtres préparés dans le paragraphe 2.I montrent que tous les composants de la formulation sont retrouvés à des températures de décompositions équivalentes.

**Tableau 5 : Résultats des analyses thermogravimétriques**

| Température de décomposition | Mélange A4 | Mélange B4 |
|---|---|---|
| ~ 305°C | ~ 4,2% | ~ 4,5% |
| ~ 400°C | ~ 91,8% | ~ 90,5% |
| Résidus | ~ 0,53% | ~ 0,16% |

| Température de décomposition | Mélange A5 | Mélange B5 |
|---|---|---|
| ~ 300°C | ~ 3,8% | ~ 5,3% |
| ~ 380°C | ~ 78,9% | ~ 78,6% |
| ~ 455°C | ~ 3,6% | ~ 3,6% |
| ~ 665°C | ~ 5,4% | ~ 4,9% |
| Résidus | ~ 6,8% | ~ 6,5% |

Les mélange-maîtres **A4** et **A5** (état de la technique) possèdent des pertes de masses équivalentes à des températures similaires à celles des mélanges-maître **B4** et **B5** obtenus selon l'invention.

L'utilisation d'une solution enzymatique formulée selon l'état de la technique ou non-formulée selon l'invention lors de la préparation des mélange-maîtres n'a pas d'impact sur les pertes de masses lors de l'analyse thermogravimétrique.

### Composition des films

Les films ont été préparés avec les mélanges-maîtres polymère support et enzymes **A4** et **B4** et un grade à base de PLA et PBAT commercialisé sous la référence ECOVIO F2223 par la société BASF et nommé « Compound 1 » dans les exemples ci-dessous.

Les compositions de ces différents films sont répertoriées dans le tableau 9.

**Tableau 6 : Récapitulatif des films monocouches réalisés**

| | Composition film | Teneur globale en enzyme (%) |
|---|---|---|
| Film 7 | Compound 1 + Mélange A4 | ∼ 0,097% |
| Film 8 | Compound 1 + Mélange B4 | |

Le film 7 sert de référence de l'état de la technique pour le film 8 de l'invention.

Le procédé de fabrication du mélange n'a pas d'impact sur le process d'extrusion gonflage. Les paramètres process de l'extrusion gonflage restent identiques entre le film de la technique et de l'invention. Quel que soit le procédé de fabrication utilisé, l'aspect des films est identique.

### Propriétés mécaniques des films 7 et 8

Les propriétés mécaniques mesurées sont présentées dans le tableau 7. Les valeurs indiquées représentent la moyenne de l'ensemble des mesures réalisées.

**Tableaux 7 : Caractérisation des propriétés mécaniques des films 7 et 8**

| | | | |
|---|---|---|---|
| Film | | 7 | 8 |
| densité film (g/cm³) film | | 1,25 | 1,25 |
| épaisseur (µm) | | 17,3 | 16,8 |

| Propriétés | *Sens de mesure* | | |
|---|---|---|---|
| Contrainte à la rupture (%) | SL | 100% | +27% |
| | ST | 100% | +24% |
| Allongement à la rupture (%) | SL | 100% | +14% |
| | ST | 100% | +24% |
| Module Young (%) | SL | 100% | +11% |
| | ST | 100% | +15% |
| Résistance à la déchirure (%) | SL | 100% | +4,2% |
| | ST | 100% | +5,2% |
| Résistance à la perforation - Dart test (%) | | 100% | +80% |

| | | | |
|---|---|---|---|
| (avec SL = Sens longitudinal du film et ST = Sens transversal du film) | | | |

Les propriétés mécaniques mesurées montrent que le film 8 obtenu avec le mélange maître **B4** selon l'invention a des propriétés mécaniques supérieures au film 7 obtenu avec le mélange maître **A4** selon l'état de la technique.

## Revendications

1. Procédé de préparation d'un mélange maître comprenant un polysaccharide, des enzymes capables de dégrader des polyesters et un polymère support dans un mélangeur, **caractérisé en ce que** ledit procédé comprend les étapes suivantes de :
a) introduction séparée et simultanée d'une formulation enzymatique liquide, du polysaccharide et du polymère support,
b) leur mélange à une température à laquelle le polymère support est partiellement ou totalement fondu, et de
c) récupération du mélange maître après mélange.

2. Procédé selon la revendication 1, **caractérisé en ce que** le polysaccharide est choisi parmi les dérivés de l'amidon, les gommes naturelles, les polysaccharides solubles de soja, les extraits marins et les polysaccharides microbiens ou animaux, ou leur mélange en toutes proportions.

3. Procédé selon l'une des revendications 1 ou 2, **caractérisé en ce que** le polysaccharide est la gomme arabique.

4. Procédé selon l'une des revendications 1 à 3, **caractérisé en ce que** les enzymes sont ajoutées sous forme de solution aqueuse.

5. Procédé selon l'une des revendications 1 à 4, **caractérisé en ce que** la formulation enzymatique comprend 0,01 à 70% en poids d'enzymes.

6. Procédé selon l'une des revendications 1 à 5, **caractérisé en ce que** les enzymes sont choisis parmi les enzymes capables de dégrader les polyesters choisis parmi les dépolymérases, les estérases, les lipases, les cutinases, les carboxylesterases, les protéases et les polyestérases.

7. Procédé selon l'une des revendications 1 à 7, **caractérisé en ce que** le polymère support est choisi parmi le polycaprolactone (PCL), le poly butylene succinate (PBS), le poly butylene succinate adipate (PBSA), le poly butylene adipate terephtalate (PBAT), le polydioxanone (PDS), le polyhydroxyalkanoate (PHA) et l'acide polylactique (PLA) et leurs mélanges, de préférence le polycaprolactone (PCL).

8. Procédé selon l'une des revendications 1 à 7, **caractérisé en ce que** le mélange à l'étape b) se fait entre 10 et 35 secondes, avantageusement entre 15 et 35 secondes, en particulier pendant environ 20 secondes, environ 25 secondes ou environ 30 secondes.

9. Procédé selon l'une des revendications 1 à 8, **caractérisé en ce qu'**il comprend l'addition simultanée d'une charge minérale à l'étape a), en particulier du carbonate de calcium.

10. Procédé selon l'une des revendications 1 à 9, **caractérisé en ce que** le mélangeur est une extrudeuse.

11. Procédé selon la revendication 10, **caractérisé en ce que** l'extrudeuse comprend au moins 3 zones, une zone de tête où sont introduits les premiers composants, une zone de mélange et une zone de sortie par laquelle le mélange maître est récupéré, avec les étapes a) à c) suivantes :
a) l'introduction séparée et simultanée d'une formulation enzymatique liquide, d'un polysaccharide, d'un polymère support et éventuellement une charge minérale en zone de tête, et leur mélange à une température inférieure ou égale à la température de fusion du polymère support,
b) le mélange des composants dans la zone de mélange à une température à laquelle le polymère support est partiellement ou totalement fondu, et
c) la récupération du mélange maître en sortie de l'extrudeuse.

12. Procédé selon l'une des revendications 1 à 11, **caractérisé en ce que** le mélange maître est obtenu à l'étape c) sous forme de granulés.

13. Procédé selon la revendication 12, **caractérisé en ce que** les granulés de mélange maître sont séchés.

14. Procédé selon l'une des revendications 1 à 13, **caractérisé en ce que** la formulation du mélange maître contient
- 60 à 90% de polymère support,
- 10 à 20% de solution enzymatique,
- 2 à 15% de polysaccharide,
- 0 à 20% de charge minérale.

15. Procédé de préparation d'un article de matière plastique ou d'un pré-mélange comprenant un polymère susceptible d'être dégradé par des enzymes et des enzymes capables de dégrader le dit polymère, **caractérisé en ce qu'**il comprend une étape de préparation d'un mélange maître selon l'une des revendications 1 à 14, et une étape de mélange du mélange maître précédemment préparé avec le polymère , les enzymes du mélange maître étant capable de dégrader le dit polymère de l'article de matière plastique ou du pré-mélange.

16. Procédé selon la revendication 15, **caractérisé en ce que** le polymère susceptible d'être dégradé par des enzymes de l'article en matière plastique ou du pré-mélange est de l'acide polylactique (PLA).

## Patentansprüche

1. Verfahren zur Herstellung eines Masterbatches, der ein Polysaccharid, Enzyme, die in der Lage sind, Polyester abzubauen, und ein Trägerpolymer umfasst, in einem Mischer, **dadurch gekennzeichnet, dass** das Verfahren die folgenden Schritte umfasst:
a) getrenntes und gleichzeitiges Einführen einer flüssigen Enzymformulierung, des Polysaccharids und des Trägerpolymers,
b) ihr Mischen bei einer Temperatur, bei der das Trägerpolymer teilweise oder vollständig geschmolzen ist, und
c) Gewinnen des Masterbatches nach dem Mischen.

2. Verfahren nach Anspruch 1, **dadurch gekennzeichnet, dass** das Polysaccharid ausgewählt ist aus Stärkederivaten, natürlichen Gummen, löslichen Sojapolysacchariden, Meeresextrakten und mikrobiellen oder tierischen Polysacchariden oder deren Mischung in jeglichen Verhältnissen.

3. Verfahren nach einem der Ansprüche 1 oder 2, **dadurch gekennzeichnet, dass** das Polysaccharid Gummi arabicum ist.

4. Verfahren nach einem der Ansprüche 1 bis 3, **dadurch gekennzeichnet, dass** die Enzyme in Form einer wässrigen Lösung zugegeben werden.

5. Verfahren nach einem der Ansprüche 1 bis 4, **dadurch gekennzeichnet, dass** die Enzymformulierung 0,01 bis 70 Gew.-% Enzyme umfasst.

6. Verfahren nach einem der Ansprüche 1 bis 5, **dadurch gekennzeichnet, dass** die Enzyme ausgewählt sind aus Enzymen, die in der Lage sind, Polyester abzubauen, ausgewählt aus Depolymerasen, Esterasen, Lipasen, Cutinasen, Carboxylesterasen, Proteasen und Polyesterasen.

7. Verfahren nach einem der Ansprüche 1 bis 6, **dadurch gekennzeichnet, dass** das Trägerpolymer ausgewählt ist aus Polycaprolacton (PCL), Polybutylensuccinat (PBS), Polybutylensuccinat-Adipat (PBSA), Polybutylenadipat-Terephtalat (PBAT), Polydioxanon (PDS), Polyhydroxyalkanoat (PHA) und Polymilchsäure (PLA) und deren Mischungen, vorzugsweise Polycaprolacton (PCL).

8. Verfahren nach einem der Ansprüche 1 bis 7, **dadurch gekennzeichnet, dass** das Mischen in Schritt b) zwischen 10 und 35 Sekunden, vorteilhafterweise zwischen 15 und 35 Sekunden, insbesondere während etwa 20 Sekunden, etwa 25 Sekunden oder etwa 30 Sekunden erfolgt.

9. Verfahren nach einem der Ansprüche 1 bis 8, **dadurch gekennzeichnet, dass** es in Schritt a) das gleichzeitige Hinzufügen einer Mineralfüllung, insbesondere Calciumcarbonat, umfasst.

10. Verfahren nach einem der Ansprüche 1 bis 9, **dadurch gekennzeichnet, dass** der Mischer ein Extruder ist.

11. Verfahren nach Anspruch 10, **dadurch gekennzeichnet, dass** der Extruder mindestens 3 Bereiche umfasst, einen Kopfbereich, in den die ersten Komponenten eingeführt werden, einen Mischbereich und einen Ausgangsbereich, durch den der Masterbatch gewonnen wird, mit den folgenden Schritten a) bis c):
a) getrenntes und gleichzeitiges Einführen einer flüssigen Enzymformulierung, eines Polysaccharids, eines Trägerpolymers und gegebenenfalls einer Mineralfüllung in den Kopfbereich und ihr Mischen bei einer Temperatur, die kleiner oder gleich der Schmelztemperatur des Trägerpolymers ist,
b) Mischen der Komponenten im Mischbereich bei einer Temperatur, bei der das Trägerpolymer teilweise oder vollständig geschmolzen ist, und
c) Gewinnen des Masterbatches am Ausgang des Extruders.

12. Verfahren nach einem der Ansprüche 1 bis 11, **dadurch gekennzeichnet, dass** der Masterbatch in Schritt c) in Form von Granulat erhalten wird.

13. Verfahren nach Anspruch 12, **dadurch gekennzeichnet, dass** das Granulat des Masterbatches getrocknet wird.

14. Verfahren nach einem der Ansprüche 1 bis 13, **dadurch gekennzeichnet, dass** die Formulierung des Masterbatches Folgendes enthält
- 60 bis 90 % Trägerpolymer,
- 10 bis 20 % Enzymlösung,
- 2 bis 15 % Polysaccharid,
- 0 bis 20 % Mineralfüllung.

15. Verfahren zur Herstellung eines Kunststoffartikels oder einer Vormischung, die ein Polymer, das durch Enzyme abgebaut werden kann, und Enzyme umfasst, die in der Lage sind, das Polymer abzubauen, **dadurch gekennzeichnet, dass** es einen Schritt des Herstellens eines Masterbatches nach einem der Ansprüche 1 bis 14 und einen Schritt des Mischens des zuvor hergestellten Masterbatches mit dem Polymer umfasst, wobei die Enzyme des Masterbatches in der Lage sind, das Polymer des Kunststoffartikels oder der Vormischung abzubauen.

16. Verfahren nach Anspruch 15, **dadurch gekennzeichnet, dass** das Polymer des Kunststoffartikels oder der Vormischung, das durch Enzyme abgebaut werden kann, Polymilchsäure (PLA) ist.

## Claims

1. A method for preparing a masterbatch comprising a polysaccharide, enzymes capable of degrading polyesters and a support polymer in a mixer,
**characterised in that** said method comprises the following steps:
a) separately and simultaneously introducing a liquid enzymatic formulation, polysaccharide and the support polymer,
b) mixing them at a temperature at which the support polymer is partially or totally molten, and
c) recovering the masterbatch after mixing.

2. The method according to claim 1, **characterised in that** the polysaccharide is selected from among starch derivatives, natural gums, soluble soybean polysaccharides, marine extracts and microbial and animal polysaccharides, or mixtures thereof in any proportions.

3. The method according to one of claims 1 or 2, **characterised in that** the polysaccharide consists of gum arabic.

4. The method according to one of claims 1 to 3, **characterised in that** the enzymes are added in the form of an aqueous solution.

5. The method according to one of claims 1 to 4, **characterised in that** the enzymatic formulation comprises 0.01 to 70% by weight of enzymes.

6. The method according to one of claims 1 to 5, **characterised in that** the enzymes are selected from among enzymes capable of degrading the polyesters selected from among depolymerases, esterases, lipases, cutinases, carboxylesterases, proteases and polyesterases.

7. The method according to one of claims 1 to 6, **characterised in that** the support polymer is selected from among polycaprolactone (PCL), polybutylene succinate (PBS), polybutylene succinate adipate (PBSA), polybutylene adipate terephthalate (PBAT), polydioxanone (PDS), polyhydroxyalkanoate (PHA) and polylactic acid (PLA) and mixtures thereof, preferably polycaprolactone (PCL).

8. The method according to one of claims 1 to 7, **characterised in that** mixing in step b) lasts between 10 and 35 seconds, advantageously between 15 and 35 seconds, in particular for about 20 seconds, for about 25 seconds or for about 30 seconds.

9. The method according to one of claims 1 to 8, **characterised in that** it comprises simultaneously adding a mineral filler in step a), in particular calcium carbonate.

10. The method according to one of claims 1 to 9, **characterised in that** the mixer is an extruder.

11. The method according to claim 10, **characterised in that** the extruder comprises at least 3 areas, a head area where the first components are introduced, a mixing area and an outlet area through which the masterbatch is recovered, with the following steps a) to c):
a) separately and simultaneously introducing a liquid enzymatic formulation, a polysaccharide and a support polymer and possibly a mineral filler in the head area, and mixing them at a temperature lower than or equal to the melting point of the support polymer,
b) mixing the components in the mixing area at a temperature at which the support polymer is partially or totally molten, and
c) recovering the masterbatch at the outlet of the extruder.

12. The method according to one of claims 1 to 11, **characterised in that** the masterbatch is obtained in step c) in the form of granules.

13. The method according to claim 12, **characterised in that** the masterbatch mixture are dried.

14. The method according to one of claims 1 to 13, **characterised in that** the formulation of the masterbatch contains
- 60 to 90% of a support polymer,
- 10 to 20% of an enzymatic solution,
- 2 to 15% of polysaccharide,
- 0 to 20% of mineral filler.

15. A method for preparing a plastic item or a pre-mixture comprising a polymer that is able to be degraded by enzymes and enzymes capable of degrading said polymer, **characterised in that** it comprises a step of preparing a masterbatch according to one of claims 1 to 14, and a step of mixing the masterbatch previously prepared with the polymer, the enzymes of the masterbatch being capable of degrading said polymer of the plastic item or of the pre-mixture.

16. The method according to claim 15, **characterised in that** the polymer that could be degraded by enzymes of the plastic item or of the pre-mixture is polylactic acid (PLA).
